Europäisches Patentamt

European Patent Office

Office européen des brevets

(11) Numéro de publication: **0 080 405**
**B1**

(12) **FASCICULE DE BREVET EUROPEEN**

(45) Date de publication du fascicule du brevet: **10.06.87**

(51) Int. Cl.⁴: **A 61 F 2/00,** A 61 L 25/00,
C 08 L 33/08, A 61 K 6/08

(21) Numéro de dépôt: **82402093.7**

(22) Date de dépôt: **17.11.82**

(54) **Compositions pour ciment chirurgical à base d'au moins un monomère acrylique et d'au moins un polymère acrylique.**

(30) Priorité: **20.11.81 FR 8121732**

(43) Date de publication de la demande:
**01.06.83 Bulletin 83/22**

(45) Mention de la délivrance du brevet:
**10.06.87 Bulletin 87/24**

(84) Etats contractants désignés:
**AT BE CH DE GB IT LI LU NL SE**

(56) Documents cités:
**FR-A-2 277 856**
**FR-A-2 418 253**

(73) Titulaire: **ALTULOR S.A.**
**Tour Gan - Place de l'Iris**
**F-92082 Paris la Defense (FR)**
(73) Titulaire: **C L L S.A.R.L.**
**17, rue de Favreuse**
**F-91430 Vauhallan (FR)**

(72) Inventeur: **Evrard, Paul**
**16 Impasse de la Couture**
**F-78112 Fourqueux (FR)**
Inventeur: **Lahille, Michel**
**17, rue de Favreuse**
**F-91430 Vauhallan (FR)**
Inventeur: **Avenel, Michel**
**107 Le Pré Langlois**
**F-27140 Gisors (FR)**

(74) Mandataire: **Dubost, Thierry**
**SOCIETE CHIMIQUE DES CHARBONNAGES**
**Service Propriété Industrielle B.P. 57**
**F-62670 Mazingarbe (FR)**

Courier Press, Leamington Spa, England.

## 0 080 405

**Description**

La présente invention concerne des compositions pour ciment chirurgical à base d'au moins un monomère acrylique et d'au moins un polymère acrylique.

Il est connu, en vue d'obtenir un ciment chirurgical, de mélanger une phase solide constituée d'une poudre de polyméthacrylate de méthyle et un sirop constitué d'une poudre de polyméthacrylate de méthyle en solution dans du méthacrylate de méthyle, puis de faire durcir le mélange à l'aide d'un catalyseur de polymérisation. Le catalyseur peut être présent dans la poudre de polyméthacrylate de méthyle constituant la phase solide. Ainsi le brevet françis n° 2.418.253 décrit un ciment applicable en chirurgie osseuse et en stomatologie, préparé à partir de 35 à 70% en poids d'une phase polymère acrylique solide contenant un catalyseur de polymérisation et de 30 à 65% en poids d'une phase liquide visqueuse comprenant de 15 à 35% en poids d'un polymère acrylique en solution dans au moins 65% en poids d'un monomére acrylique.

Il est d'autre part connu par le brevet français n° 2.277.856 d'obtenir une composition de moulage pour la fabrication de prothèses dentaires par mélange de 70 à 75% d'une poudre contenant 60 à 90% de polyméthacrylate de méthyle et 10 à 40% d'un copolymère méthacrylate de méthyle/acrylate de 2-éthylhexyle, et de 20 à 25% d'un liquide polymérisable (méthacrylate de méthyle seul ou additionné d'agent(s) de réticulation). Le durcissement a lieu à 100°C en présence d'un catalyseur de polymérisation. Ce brevet propose d'ajouter au mélange de 0,005 à 0,5% d'un suppresseur de pic exothermique. Le seul suppresseur de pic exothermique donné en exemple est le dipent ène utilisé à raison de 0,01% en poids par rapport au liquide polymerisable.

Les compositions selon l'art antérieur ne sont pas satisfaisantes, soit que leur durcissement soit trop exothermique comme dans le premier document cité, soit que leur temps de durcissement total après mélange à température ambiante soit trop long comme dans le second document cité.

On a maintenant trouvé une composition pour ciment chirurgical dont la durée pratique d'utilisation est remarquablement courte (généralement inférieure à 12 minutes) et dont la température maximale atteinte pendant la polymérisation est couramment inférieure à 55°C.

La présente invention a pour objet une composition pour ciment chirurgical comprenant:

— d'une part une poudre en une quantité telle qu'elle contienne de 50 à 70 parties en poids d'au moins un polymère acrylique de granulométrie comprise entre 20 et 150 μm et comprenant en outre un initiateur de polymérisation, et

— d'autre parts une phase liquide en une quantité telle qu'elle contienne de 30 à 50 parties en poids d'un mélange comprenant au moins 65% en poids d'au moins un monomère acrylique, et jusqu'à 35% en poids d'au moins un polymère acrylique, ladite phase liquide comprenant en outre au moins un activateur de polymérisation, les quantités respectives de polymère acrylique pulvérulent d'une part et du mélange monomére acrylique — polymère acrylique d'autre part étant telles que leur somme soit égale à 100 parties, caractérisée en ce que ladite phase liquide comprend en outre de 0,05 à 1% en poids, par rapport à la quantité totale de monomère acrylique, d'au moins un limiteur de chaîne choisi parmi les terpènes monocycliques di-insaturés et les terpènes bicycliques monoinsaturés.

On entend par polymére acrylique un produit obtenu par homopolymérisation d'un monomère acrylique ou par copolymérisation d'un monomère acrylique avec au moins un second monomère acrylique et/ou comonomère tel que le styrène, l'acrylonitrile, les esters vinyliques tel que l'acétate de vinyle. On entend par monomère acrylique un ester de l'acide acrylique ou de l'acide méthacrylique avec un alcool comprenant de 1 à 8 atomes de carbone. On utilise avantageusement de façon principale le méthacrylate de méthyle d'une part pour la fabrication de la poudre de polymère acrylique et d'autre part comme constituant de la phase liquide. Le polymère acrylique est préparé par polymérisation d'un monomère acrylique en présence, le cas échéant, d'au moins un autre monomère, par tout moyen connu, par exemple en suspension. Les perles obtenues de cette façon, ou bien par broyage cryogénique de polymère préparé en masse, sont tamisées de façon à ne conserver essentiellement que la fraction dont la granulométrie est comprise entre 20 et 150 μm. La granulométrie du polymère acrylique ne doit, en effet, pas être quelconque. Les particules inférieures à 20 μm conduisent à un épaississement très rapide de la composition qui retient ainsi de l'air occlus et conduit à une structure hétérogène. Les particules supérieures à 150 μm se dissolvent mal dans le monomère acrylique alors que la polymérisation de ce dernier commence, conduisant ainsi à une structure également hétérogène.

La poudre contient un homopolymère acrylique ou un copolymère acrylique ou bien un mélange d'homopolymères et/ou de copolymères acryliques. La masse moléculaire moyenne du polymère acrylique est avantageusement comprise entre 200.000 et 600.000.

La poudre contenant au moins un polymère acrylique comprend un initiateur de polymérisation connu tel qu'un peroxyde minéral ou organique ou l'azobisisobutyronitrile (AIBN) ou un photoinitiateur. On utilise avantageusement le peroxyde de benzoyle, éventuellement phlegmatisé à l'aide de 15 à 50% d'eau.

L'initiateur peur être ajouté à la poudre de polymère acrylique ou bien être déjà présent celle-ci si on en a utilisé un excès lors de la polymérisation.

Le mélange contenu dans la phase liquide comprend au moins 65% en poids d'au moins un monomère acrylique. Ledit mélange peut être uniquement constitué d'un monomère ou d'un mélange de

2

comonomères auquel on ajoute un activateur de polymérisation et au moins un terpène monocyclique diinsaturé ou bicyclique monoinsaturé.

Avantageusement le mélange forme un sirop comprenant au moins un monomère acrylique et au moins un polymère acrylique. La granulométrie du polymère à dissoudre dans le monomère n'est pas critique; il suffit de s'assurer que la solution obtenue est homogène. Le polymère utilisé est semblable ou différent de celui qui est contenu dans la poudre et sa masse moléculaire moyenne est avantageusement comprise entre 100.000 et 400.000.

La phase liquide peut également contenir un monomère polyinsaturé en faible quantité (de 0,1 à 2 parties pour 100 parties de monomère acrylique) choisi avantageusement parmi les esters de l'acide acrylique ou de l'acide méthacrylique et de polyols. Onb utilise par exemple le diméthacrylate d'éthylène-glycol, le triméthacrylate de triméthylolpropane.

L'activateur de polymérisation ajouté à la phase liquide est choisi parmi les produits connus pour accélérer les polymérisation radicalaires en présence de radicaux libres. On choisit avantageusement une amine tertiaire, de préférence comportant au moins un noyau aromatique telles les N,N-dialkylamilines et les N,N-dialkylparatoluidines, et par exemple la N,N-diméthlparatoluidine en une quantité comprise entre 1 et 5% en poids par rapport à la quantité total ede monomère contenu dans la phase liquide.

Le limiteur de chaîne est choisi parmi les terpènes monocycliques diinsaturés tels que le limonène, le 3,8-paramenthadiène, 1'α-phellandrène, le β-phellandrène, 1'α-terpinène, le β-terpinène, le δ-terpinène, le terpinolène, l'isoterpinolène et parmi les terpènes bicycliques monoinsaturés tels que le sabinène et le β-pinène.

On utilise avantageusement le terpinolène en une quantité comprise, de préférence, entre 0,2 et 0,9% en poids par rapport à la quantité totale de monomère acrylique.

On a constaté qu'il faut non seulement que le terpène présent lors de la prise du ciment chirurgical agisse comme suppresseur de pic dr façon à empêcher une exothermie préjudiciable aux tissus organiques à son contact mais également qu'il doive être présent en une quantité telle qu'il agisse réellement comme un limiteur de chaîne c'est-à-dire qu'il évite, lors de la polymérisation du monomère acrylique en présence de polymère acrylique, la formation de macromolécules à chaînes longues donc de masse moléculaire élevée. De cette façon la prise du ciment chirurgical selon la présente invention a lieu à une température qui n'excède pas 55°C lorsque le mélange de la poudre et de la phase liquide (environ 70 à 80 g de masse totale) est effectué à la température ambiante (environ 18 à 20°C) et que le durcissement a lieu à la température normale du corps humain (environ 37,2°C). Même avec une exothermie aussi peu importante il est étonnant de constater que le taux de conversion maximal du monomère est obtenu en un temps remarquablement court (généralement inférieur à 12 minutes).

On a en outre constaté que l'activateur renforce la fonction de limiteur de chaîne du terpène utilisé. Ainsi, partant d'une composition donnée il est possible de diminuer la température maximale atteinte par le mélange soit en ajoutant une quantité supplémentaire de terpène soit en ajoutant une quantité supplémentaire d'activateur. L'addition simultanée d'une quantité supplémentaire de chacun de ces constituants a pour effet de diminuer de façon encore plus sensible l'exothermie de la polymérisation. Cette constatation est particulièrement surprenante dans la mesure où l'activateur, utilisé seul, ne possède pas de fontion de limiteur de chaîne et/ou de suppresseur du pic exothermique.

La présente invention est secondairement caractérisée en ce que la phase liquide peut contenir un stabilisant en une quantité comprise entre 0,001 et 0,03% en poids par rapport à la quantité totale de monomère acrylique. Ce stabilisant a pour rôle d'empêcher la polymérisation prématurée du monomère acrylique. Il est choisi avantageusement parmi les monophénols alkyl substitués et tel que par exemple le 6-tertiobutyl-2,4-diméthylphénol.

Les compositions peuvent également comprendre d'autres ingrédients dont les fonctions sont diverses. Ainsi la poudre peut contenir de 30 à 50% en poids ou plusieurs charges inertes destinées à augmenter la résistance à la compression du ciment obtenu et à diminuer le retrait au cours de la polymérisation. On utilise par example une apatite — comme l'hydroxyapatite — la silice, l'alumine, 'a vitro-céramique, des fibres de carbone, d'aramide, de verre ou de bore, des fibres métalliques, par exemple en acier inoxydable, tantale ou titane. On peut également utiliser une charge inerte organique, insoluble dans le monomère contenu dans la phase liquide. Un exemple d'une telle charge est constitué par un homopolymère ou copolymère acrylique de masse moléculaire moyenne très élevée (généralement supérieure à 600.000) se présentant sous la forme de poudre ou de billes de granulométrie comprise entre 10 et 500 μm. La présence d'une telle charge augmente la résistance mécanique du ciment obtenu.

Les compositions selon l'invention peuvent également contenir des charges bioabsorbables, par exemple des polymères qui peuvent être résorbés dans le temps et assimilés par l'organisme. Un exemple d'une telle charge est constitué par du sérum physiologique épaissi à l'aide d'un polysaccharide (glucane, xanthane, chitine) qui, après résorption, laisse en place une structure microporeuse favorable à la réhabitation par l'os.

La poudre peut également contenir des charges opaques aux rayons X comme le sulfate de baryum ou l'oxyde de zirconium, des colorants, par exemple $Fe_2O_3$. Les compositions selon l'invention peuvent, si nécessaire, également contenir un ou plusieurs antiseptiques ou bien un ou plusierus antibiotiques, par exemple un aminoglucoside.

La composition objet de la présente invention est utilisable dans la fabrication d'un ciment chirurgical.

3

# 0 080 405

A cette fin, elle est coulée dans un espace destiné à mise en place d'une prothèse, à une température comprise entre environ 10 et 30°C, une composition telle que décrite ci-dessus.

Lors de l'utilisation, les deux constituants de la composition (la poudre d'une part et la phase liquide d'autre part) sont mélangés dans des proportions telles que les quantités relatives de polymère acrylique d'une part et de mélange contenu dans la phase liquide, d'autre part soient respectées. On obtient une pâte lisse et homogène. Cette pâte est ensuite mise en place dans l'espace laissé libre, par exemple par l'ablation du col d'un fémur, et on procède alors à la mise en place dans cette pâte de la prothèse, généralement métallique, destinée à remplacer le col du fémur. Après 8 à 10 minutes, la température au sein du ciment chirurgical atteint 22 à 25°C et la composition durcit en un temps total, depuis le mélange de la poudre et de la phase liquide, de 12 à 15 minutes en atteignant une température de 40 à 45°C.

Les compositions selon l'invention peuvent bien entendu être utilisées dans d'autres applications où la température maximale admise lors d'une intervention chirurgicale (généralement 56°C) peut être dépassée. C'est le cas par exemple de la confection des prothèses dentaires.

Les exemples suivants, non limitatifs, ont pour but d'illustrer l'invention.

## EXEMPLES 1 à 30

Dans tous ces exemples, le mode opératoire suivant a été utilisé:

| | |
|---|---|
| On prépare d'une part une poudre composée de: | |
| Polyméthacrylate de méthyle de granulométrie comprise entre 20 et 150 µm | 43 g |
| Oxyde de zirconium | 5 g |
| Peroxyde de benzoyle à 85% en poids, phlegmatisé à l'eau | 0,56 g |

On prépare d'autre part une phase liquide contenant 22 g de méthacrylate de méthyle ainsi que du terpinolène et de la N,N-diméthylparatoluidine (N,N-DMPT) selon les quantités indiquées ci-après.

Au temps t=0 la phase liquide est ajoutée à la poudre à la température de 18°C. On mélange à l'aide d'une spatule pendant 1,5 min et coule le mélange obtenu dans un moule parallélépipédique de dimensions 5 x 4,5 x 3,5 cm et on note la température maximale atteinte par le mélange.

### Exemples 1 et 2 (comparatifs)
La phase liquide ne contient pas de terpinolène. Elle contient 0,33 g (exemple 1) ou 0,99 g (exemple 2) de N,N-DMPT. Les températures maximale atteintes sont de 101°C (exemple 1) et 98°C (exemple 2).

### Exemples 3 à 30
La phase liquide contient les quantités de terpinolène et de N,N-DMPT mentionnées au tableau I. La température maximale atteinte par le mélange est indiquée également dans ce tableau I. Dans le tableau I la numérotation des exemples doit être comprise de gauche à droite.

On constate que la température atteinte par un mélange donné est inférieure à celle atteinte par un mélange contenant une teneur inférieure en terpinolène, la teneur en N,N-DMPT étant identique; elle est également inférieure à celle atteinte par un mélange contenant une tenuer inférieure en N,N-DMPT, la teneur en terpinolène étant identique.

On a constaté d'autre part que, pour une teneur en terpinolène donnée, le fait d'augmenter la teneur en N,N-DMPT conduit à un temps d'obtention de la température maximale plus court, bien que cette température maximale soit inférieure.

Ainsi dans l'exemple 24 le temps mis par le mélange pour atteindre 62,5°C est de 26 min; il n'est que de 16 min pour atteindre 42°C dans l'exemple 30.

### Exemples 31 à 42
On a utilisé les mêmes constituants qu'aux exemples 1 à 30 pour la poudre d'une part et pour la phase liquide, d'autre part.

On a utilisé un moule dont le format intérieur et l'épaisseur sont conformes à la norme française NF-S 90 430. Le moule est constitué d'une couronne cylindrique en polytétrafluoroéthylène de diamètre extérieur 90 mm, de diamètre intérieur 60 mm et d'épaisseur 6 mm, munie d'une entaille en V pour le remplissage et d'un orifice pour le passage de la sonde de mesure de la température. La couronne est emprisonnée, à l'aide de serre-joints, entre 2 flasques en aluminium de 90 mm de diamètre et 1,5 mm d'épaisseur, revêtus d'une couche de 50 µm de polytétrafluoroéthylène.

Le mélange de la poudre et de la phase liquide est effectué comme pour les exemples 1 à 30 et coulé dans le moule au bout de 3,5 min. Après une minute de repos le moule est immergé dans une enceinte thermostatée contenant du sérum physiologique maintenu à la température de 37,2°C. On note la valeur maximale atteinte par la température au cours de lay polymérisation. Le tableau II mentionne les valeurs

4

**0 080 405**

pour des mélanges ayant différentes teneurs en terpinolène et N,N-DMPT. La valeur maximale pour la température est atteinte en un temps d'autant plus court que la teneur en N,N-DMPT est plus élevée mais les écarts sont peu sensibles. Les temps s'échelonnent de 13 à 11 min après immersion du moule dans le bain thermostaté.

Exemple 43 (comparitif)

Dans les mêmes conditions de mesure que pour les exemples 31 à 42 on prépare, d'une part une poudre contenant:

| | |
|---|---|
| copolymère de méthacrylate de méthyle (90%) et de méthacrylate de butyle (10%) | 46,95 g |
| peroxyde de benzoyle à 85% en poids | 0,12 g |

et d'autre part une phase liquide contenant:

| | |
|---|---|
| méthacrylate de méthyle | 17,58 g |
| méthacrylate de butyle | 1,95 g |
| N,N-DMPT | 0,4 g |

La température maximale atteinte par le mélange de la poudre et de la phase liquide est de 65,2°C.

Exemple 44

Dans les mêmes conditions qu'à l'exemple 43 on utilise une phase liquide contenant, en outre, 0,07 g de terpinolène.

Le température maximale atteinte est de 46,1°C.

Exemple 45 (comparitif)

Dans les mêmes conditions de mesure qu'aux exemples 31 à 42, on réalise une composition comprenant:

| | |
|---|---|
| poudre: polyméthacrylate de méthyle | 43 g |
| $ZrO_2$ | 5 g |
| peroxyde de benzoyle à 85% en poids | 0,56 g |
| phase liquide: méthacrylate de méthyle | 21,8 g |
| triméthacrylate de triméthylolpropane | 0,2 g |
| N,N-DMPT | 0,48 g |

La température maximale atteinte après mélange est de 63,4°C.

Exemple 46 (comparitif)

Dans les mêmes conditions de mesure qu'aux exemples 31 à 42, on réalise une composition comprenant:

| | |
|---|---|
| poudre: polyméthacrylate de méthyle | 39,53 g |
| peroxyde de benzoyle à 85% | 0,47 g |
| phase liquide: polyméthacrylate de méthyle | 8 g |
| méthacrylate de méthyle | 18,6 g |
| N,N-DMPT | 0,4 g |

La température maximale atteinte après mélange est de 65,5°C.

5

Exemple 47

On ajoute à la phase liquide de l'exemple 45, du terpinolène (0,12 g). La valeur maximale atteinte par la température est alors de 44°C.

Exemple 48

Dans les conditions de mesure utilisées pour les exemples 1 à 30 on étudie un mélange obtenu à partir, d'une part, d'une poudre contenant:

| | |
|---|---|
| polyméthacrylate de méthyle | 42,7 g |
| $ZrO_2$ | 4,75 g |
| Peroxyde de benzoyle à 85% | 0,56 g |

et d'autre part, d'une phase liquide contenant:

| | |
|---|---|
| polyméthacrylate de méthyle | 7,2 g |
| méthacrylate de méthyle | 22,8 g |
| terpinolène | 0,06 g |
| N,N-DMPT | 0,55 g |
| 6-tertiobutyl-2,4-diméthylphénol | 0,006 g |

La température maximale atteinte est de 47°C.

Dans les mêmes conditions de mesure, la composition décrite à l'exemple 46, et qui ne contient pas de terpinolène, conduit à une température maximale de 70°C.

TABLEAU I

| Terpinolène g \ N,N-DMPT g | 0,33 | 0,44 | 0,55 | 0,66 | 0,77 | 0,88 | 0,99 | Exemples |
|---|---|---|---|---|---|---|---|---|
| 0,05 | 88°C | 86°C | 82°C | 78°C | 76°C | 74,5°C | 72°C | 3 à 9 |
| 0,10 | 79°C | 78°C | 71°C | 70°C | 66°C | 64°C | 63°C | 10 à 16 |
| 0,15 | 721,5°C | 69°C | 63°C | 62,5°C | 56°C | 54°C | 53°C | 17 à 23 |
| 0,20 | 62,5°C | 59°C | 56°C | 54°C | 52°C | 48°C | 42°C | 24 à 30 |

# 0 080 405

TABLEAU II

| N,N-DMPT g / Terpinolène g | 0,44 | 0,66 | 0,88 | 1,10 | Exemples |
|---|---|---|---|---|---|
| 0,05 | 48,0°C | 46,0°C | 44,7°C | 43,3°C | 31 à 34 |
| 0,10 | 44,3°C | 43,1°C | 42,8°C | 441,0°C | 35 à 38 |
| 0,20 | 42,0°C | 41,5°C | 41,3°C | 40,5°C | 39 à 42 |

**Revendications**

1. Composition pour ciment chirurgical comprenant:
— d'une part une poudre en une quantité telle qu'elle contienne de 50 à 70 parties en poids d'au moins un polymère acrylique de granulométrie comprise entre 20 et 150 µm et comprenant en outre un initiateur de polymérisation, et
— d'autre part une phase liquide en une quantité telle qu'elle contienne de 30 à 50 parties en poids d'un mélange comprenant au moins 65% en poids d'au moins un monomère acrylique, et jusqu'a 35% en poids d'au moins un polymère acrylique, ladite phase liquide comprenant en outre de 1 à 5% en poids d'au moins un activateur de polymérisation, par rapport à la quantité totale de monomère acrylique, les quantités respectives de polymère acrylique pulvérulent d'une part et du mélange monomère acrylique-polymère acrylique d'autre part étant telles que leur somme soit égale à 100 parties, caractérisée en ce que ladite phase liquide comprend en outre de 0,05 à 1% en poids, par rapport à la quantité totale de monomère acrylique, d'au moins un limiteur de chaîne choisi parmi les terpènes monocycliques diinsaturé et les terpènes bicycliques monoinsaturés.

2. Composition selon la revendication 1 caractérisée en ce que l'activateur est la N,N-diméthylparatoluidine.

3. Composition selon l'une des revendications 1 et 2 caractérisée en ce que le limiteur de chaîne est le terpinolène.

4. Composition selon l'une des revendications 1 à 3 caractérisée en ce que la phase liquide contient, en outre, un stabilisant en une quantité comprise entre 0,001 et 0,03% en poids rapport à la quantité totale de monomère acrylique.

5. Composition selon la revendication 4 caractérisée en ce que la stabilisant est choisi parmi les monophénols alkyl substitués.

6. Composition selon la revendication 5 caractérisée en ce que la stabilisant est le 6-tertiobutyl-2,4-diméthylphénol.

**Patentansprüche**

1. Zusammensetzung für chirurgischen Zement enthaltend:
— einerseits ein Pulver in einer 50 bis 70 Gewichtsteile der Zusammensetzung ausmachenden Menge mindestens eines Acrylpolymers einer Korngröße zwischen 20 und 150 µm, wobei das Pulver weiters einen Polymerisationsinitiator enthält, und
— anderseits eine flüssige Phase in einer 30 bis 50 Gewichtsteile der Zusammensetzung ausmachenden Menge einer Mischung, die mindestens 65 Gew.-% mindestens eines Acrylmonomers und bis zu 35 Gew.-% mindestens eines Acrylpolymers enthält, wobei die genannte flüssige Phase weiters 1 bis 5 Gew.-% bezogen auf die Gesamtmenge an Acrylmonomer, mindestens eines Polymerisationsaktivators enthält, und wobei die betreffenden Mengen des pulverförmigen Acrylpolymers einerseits und der Acrylmonomer/Acrylpolymer-Mischung anderseits so sind, daß ihre Summe 100 Teile ausmacht, dadurch gekennzeichnet, daß die flüssige Phase weiters 0,05 bis 1 Gew.-%, bezogen auf die Gesamtmenge an Acrylmonomer, mindestens eines Kettenbegrenzers enthält, der ausgewählt ist aus der Gruppe bestehend aus den diungesättigten monocyclischen Terpenen und den monoungesättigten bicyclischen Terpenen.

2. Zusammensetzung nach Anspruch 1, dadurch gekennzeichnet, daß der Aktivator N,N-Dimethylparatoluidin ist.

3. Zusammensetzung nach Anspruch 1 oder 2, dadurch gekennzeichnet, daß der Kettenbegrenzer Terpinolen ist.

4. Zusammensetzung nach einem der Ansprüche 1 bis 3, dadurch gekennzeichnet, daß die flüssige Phase weiters einen Stabilisator in einer Menge zwischen 0,001 und 0,03 Gew.-% in bezug auf die Gesamtmenge an Acrylmonomer enthält.

5. Zusammensetzung nach Anspruch 4, dadurch gekennzeichnet, daß der Stabilisator ausgewählt ist aus den substituierten Alkylmonophenolen.

6. Zusammensetzung nach Anspruch 5, dadurch gekennzeichnet, daß der Stabilisator 6-tert.-Butyl-2,4-dimethylphenol ist.

**Claims**

1. A surgical cement composition comprising:
on the one hand a powder in such amount that it comprises from 50 to 70 parts by weight of at least one acrylic polymer having a particle size between 20 and 150 µm and further comprising a polymerization initiator, and
on the other hand a liquid phase in such amount that it comprises from 30 to 50 parts by weight of a mixture comprising at least 65 weight percent of at least one acrylic monomer and up to 35 weight percent of at least one acrylic polymer, the said liquid phase further comprising from 1 to 5 weight percent of at least one polymerization activator, the respective amounts of the pulverulent acrylic polymer on the one hand being such that their total be equal to 100 parts, characterized in that the said liquid phase further comprises from 0.05 to 1 weight percent, with respect to the total amount of acrylic monomer, of at least one chain stopping agent selected from diunsaturated monocyclic terpenes and monounsaturated bicyclic terpenes.

2. A composition according to claim 1, characterized in that the activator is N,N-dimethyl-para-toluidine.

3. A composition according to one of claims 1 and 2, characterized in that the claim stopping agent is terpinolene.

4. A composition according to one of claims 1 to 3, characterized in that the liquid phase further comprises from 0.001 to 0.03 weight percent, relative to the total amount of acrylic monomer, of a stabilizer.

5. A composition according to claim 4, characterized in that the stabilizer is selected from alkylsubstituted monophenols.

6. A composition according to claim 5, characterized in that the stabilizer is 6-tertbutyl-2,4-dimethylphenol.